# EUROPEAN PATENT APPLICATION

(11) **EP 3 714 875 A1**
(43) Date of publication of application: **30.09.2020**
(21) Application number: 20173457.1
(22) Date of filing: 14.11.2014
(51) Int. Cl.: A61K 9/00, A61K 31/573, A61P 27/02, A61K 47/34

(54) **METHODS OF TREATMENT OF OCULAR CONDITIONS WITH A SUSTAINED DRUG DELIVERY IMPLANT**

(30) Priority: 15.11.2013 US 201361904887 P
(62) Divisional of application: 14805768.0
(71) Applicant: ALLERGAN, INC., Irvine, CA 92612 (US)
(72) Inventor: Shiah, Jane-Guo, Irvine, CA California 92620 (US); Bhagat, Rahul, Irvine, CA California 92620 (US); Blanda, Wendy M., Tustin, CA California 92780 (US); Nivaggioli, Thierry, Atherton, CA California 94027 (US); Peng, Lin, South San Francisco, CA California 94080 (US); Chou, David, Palo Alto, CA California 94306 (US); Weber, David A., Danville, CA California 94506-6004 (US)
(74) Representative: Hoffmann Eitle

(57) **Abstract**

Intraocular implants may include a corticosteroid and a biodegradable polymer associated with the corticosteroid to facilitate the release of the corticosteroid into an eye for a period of time. In some embodiments, ocular conditions, such as diabetic macular edema can be treated through administration of an intraocular implant including a corticosteroid and a biodegradable polymer associated with the corticosteroid to the eye of a human at a frequency of about once every six months to about once a year.

## Description

### CROSS-REFERENCE TO RELATED APPLICATIONS

The present application claims priority to U.S. Provisional Patent Application No. 61/904,887, filed on November 15, 2013, the entire content of which is incorporated herein by reference.

### BACKGROUND

### Field

The disclosure of the present application generally relates to drug delivery implants, and more specifically, method for treating ocular conditions using drug delivery implants.

### Description of the Related Art

Macular edema ("ME") is an ocular condition that can result in a swelling of the macula. The edema is caused by fluid leaking from retinal blood vessels. Blood leaks out of the weak vessel walls into a very small area of the macula which is rich in cones, the nerve endings that detect color and from which daytime vision depends. Blurring then occurs in the middle or just to the side of the central visual field. Visual loss can progress over a period of months. Retinal blood vessel obstruction, eye inflammation, and age-related macular degeneration have all been associated with macular edema. The macula may also be affected by swelling following cataract extraction. Symptoms of ME include blurred central vision, distorted vision, vision tinted pink and light sensitivity. Causes of ME can include retinal vein occlusion, macular degeneration, diabetic macular leakage, eye inflammation, idiopathic central serous chorioretinopathy, anterior or posterior uveitis, pars planitis, retinitis pigmentosa, radiation retinopathy, posterior vitreous detachment, epiretinal membrane formation, idiopathic juxtafoveal retinal telangiectasia, Nd:YAG capsulotomy or iridotomy. Some patients with ME may have a history of use of topical epinephrine or prostaglandin analogs for glaucoma.

Macular edema involves the breakdown of the inner blood retinal barrier at the level of the capillary endothelium, resulting in abnormal retinal vascular permeability and leakage into the adjacent retinal tissues. The macula becomes thickened due to fluid accumulation resulting in significant disturbances in visual acuity.

Macular edema may occur in diseases causing cumulative injury over many years, such as diabetic retinopathy, or as a result of more acute events, such as central retinal vein occlusion or branch retinal vein occlusion.

Diabetic retinopathy is a frequent microvascular complication of diabetes types 1 and 2 and represents the leading cause of blindness in the world. Diabetes-related central vision loss can arise either from microvascular occlusion (mascular ischemia) or from microvascular leakage due to breakdown of the inner blood-retinal barrier (BRB), leading to thickening or swelling of the macula (macular edema). Diabetic macular edema (DME) affects an estimated 21 million individuals worldwide.

Several treatment options have emerged that offer to improve visual acuity, including intravitreal anti-vascular endothelial growth factor (anti-VEGF) agents and laser photocoagulation. However, these treatment options have some drawbacks and do not work effectively for all patients.

### SUMMARY

The present disclosure is concerned with and directed to implants and methods for the treatment of an ocular condition, such as macular edema, including diabetic macular edema ("DME"). In some embodiments, the implant can contain a corticosteroid. In some embodiments, the corticosteroid is dexamethasone.

An ocular condition can include a disease, aliment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball. An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves, the conjunctiva, the cornea, the conjunctiva, the anterior chamber, the iris, the posterior chamber (behind the retina but in front of the posterior wall of the lens capsule), the lens or the lens capsule and blood vessels and nerve which vascularize or innervate an anterior ocular region or site. A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

A posterior ocular condition can include a disease, ailment or condition, such as for example, macular degeneration (such as non-exudative age related macular degeneration and exudative age related macular degeneration); choroidal neovascularization; acute macular neuroretinopathy; macular edema (such as cystoid macular edema and diabetic macular edema); Behcet's disease, retinal disorders, diabetic retinopathy (including proliferative diabetic retinopathy); retinal arterial occlusive disease; central retinal vein occlusion; uveitic retinal disease; retinal detachment; ocular trauma which affects a posterior ocular site or location; a posterior ocular condition caused by or influenced by an ocular laser treatment; posterior ocular conditions caused by or influenced by a photodynamic therapy; photocoagulation; radiation retinopathy; epiretinal membrane disorders; branch retinal vein occlusion; anterior ischemic optic neuropathy; non-retinopathy diabetic retinal dysfunction, retinitis pigmentosa and glaucoma.

An anterior ocular condition can include a disease, ailment or condition, such as for example, aphakia; pseudophakia; astigmatism; blepharospasm; cataract; conjunctival diseases; conjunctivitis; corneal diseases; corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

Potent corticosteroids such as dexamethasone suppress inflammation by inhibiting edema, fibrin deposition, capillary leakage and phagocytic migration, all key features of the inflammatory response. Corticosteroids prevent the release of prostaglandins, some of which have been identified as mediators of cystoid macular edema.

By delivering a drug, such as a corticosteroid, directly into the vitreous cavity, blood eye barriers can be circumvented and intraocular therapeutic levels can be achieved with minimal risk of systemic toxicity. This route of administration typically results in a short half-life unless the drug can be delivered using a formulation capable of providing sustained release.

Consequently, a biodegradable implant for delivering a therapeutic agent to an ocular region, such as the vitreous may provide significant medical benefit for patients afflicted with a medical condition of the eye, such as diabetic macular edema.

According to an embodiment a method for treating diabetic macular edema includes injecting a bioerodible implant into the vitreous of a human in need thereof at a frequency of once every about six months to once every about nine months. The bioerodible implant can include a continuous, double extruded rod that can include an active agent homogeneously dispersed within a biodegradable polymer matrix. The biodegradable polymer matrix can include a mixture of poly(D,L-lactide-co-glycolide) (PLGA) having hydrophilic end groups and poly(D,L-lactide-co-glycolide) (PLGA) having hydrophobic end groups. The bioerodible implant can be sized for implantation in an ocular region. In some embodiments, the active agent is a corticosteroid. The method can be therapeutically effective to treat DME In some embodiments, the active agent is dexamethasone. In some embodiments, the macular edema is diabetic macular edema. In some embodiments, the dexamethasone is present in the bioerodible implant in an amount of 60% by weight, based on the total weight of the bioerodible implant. In some embodiments, the PLGA having hydrophobic end groups is present in the bioerodible implant in an amount of 10% by weight, based on the total weight of the bioerodible implant. In some embodiments, the PLGA having hydrophilic end groups is present in the bioerodible implant in an amount of 30% by weight, based on the total weight of the bioerodible implant. According to an embodiment, the human has a pseudophakic lens. According to another embodiment, the human has a phakic lens.

### BRIEF DESCRIPTION OF THE FIGURES

These and other features will now be described with reference to the drawings summarized below. These drawings and the associated description are provided to illustrate one or more embodiments and not to limit the scope of the invention.
Figure 1 illustrates a bar graph comparing the proportion of DME patients having a BCVA improvement of greater than or equal to 15 letters in patient groups receiving example embodiment bioerodible implants according to example embodiment methods disclosed herein.
Figure 2 illustrates a bar graph comparing the proportion of DME patients having a BCVA improvement of greater than or equal to 20 letters in patient groups receiving example embodiment bioerodible implants according to example embodiment methods disclosed herein.
Figure 3 illustrates a line graph comparing the mean change in BCVA between different DME patient groups receiving example embodiment bioerodible implants according to example embodiment methods disclosed herein.
Figure 4 illustrates a bar graph comparing the mean average decrease from baseline of CSRT in DME patient groups receiving example embodiment bioerodible implants according to example embodiment methods disclosed herein.
Figure 5 illustrates a bar graph comparing the proportion of DME patients having a BCVA improvement of greater than or equal to 15 letters in patient groups receiving example embodiment bioerodible implants according to example embodiment methods disclosed herein.
Figure 6 shows a table listing common adverse events occurring during a study according to the examples.
Figure 7 shows a table listing ocular surgeries performed to correct IOP during a study according to the examples.

### DETAILED DESCRIPTION

### Definitions

The following terms as used herein have the following meanings:
"Active agent" and "drug" are used interchangeably and refer to any substance used to treat an ocular condition.
"Bioerodible polymer" means a polymer which degrades *in vivo,* and wherein erosion of the polymer over time is required to achieve the active agent release kinetics according to the present invention. Thus, hydrogels such as methylcellulose which act to release drug through polymer swelling are specifically excluded from the term "bioerodible (or biodegradable) polymer". The words "bioerodible" and "biodegradable" are synonymous and are used interchangeably herein.
"Injury" or "damage" are interchangeable and refer to the cellular and morphological manifestations and symptoms resulting from an inflammatory-mediated condition, such as, for example, inflammation.
"Ocular condition" means a disease, aliment or condition which affects or involves the eye or one or the parts or regions of the eye, such as a retinal disease. The eye includes the eyeball and the tissues and fluids which constitute the eyeball, the periocular muscles (such as the oblique and rectus muscles) and the portion of the optic nerve which is within or adjacent to the eyeball. : "Ocular condition" is synonymous with "medical condition of the eye"
"Plurality" means two or more.
"Posterior ocular condition" means a disease, ailment or condition which affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerve which vascularize or innervate a posterior ocular region or site.
"Steroidal anti-inflammatory agent" and "glucocorticoid" are used interchangeably herein, and are meant to include steroidal agents, compounds or drugs which reduce inflammation when administered at a therapeutically effective level.
"Suitable for insertion (or implantation) in (or into) an ocular region or site" with regard to an implant, means an implant which has a size (dimensions) such that it can be inserted or implanted without causing excessive tissue damage and without unduly physically interfering with the existing vision of the patient into which the implant is implanted or inserted.
"Therapeutic levels" or "therapeutic amount" means an amount or a concentration of an active agent that has been locally delivered to an ocular region that is appropriate to safely treat an ocular condition so as to reduce or prevent a symptom of an ocular condition.

According to some embodiments, a bioerodible implant for treating a medical condition of the eye comprises an active agent dispersed within a biodegradable polymer matrix. Example bioerodible implants and methods of making such implants are described in U.S. Patent No. 8,034,370, U.S. Patent No. 8,242,099, U.S. Patent No. 7,767,223, and U.S. Patent No. 8,257,7300, the entirety of all the aforementioned patents are incorporated herein by reference.

The active agent can be selected from the group consisting of ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, anti-inflammatory agents, steroids (such as a steroidal anti-inflammatory agent), antihypertensives, pressors, antibacterials, antivirals, antifungals, antiprotozoals, anti-infective agents, antitumor agents, antimetabolites, and antiangiogenic agents. Thus, the active agent can be cortisone, dexamethasone, fluocinolone, hydrocortisone, methylprednisolone, prednisolone, prednisone, triamcinolone, and any derivative thereof.

The bioerodible implant is sized for implantation in an ocular region. The ocular region can be any one or more of the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

A method for making a bioerodible implant for treating a medical condition of the eye can include a plurality of extrusions of a biodegradable polymer. This method can also comprise the step of milling the biodegradable polymer prior to the extrusion. The biodegradable polymer can be a poly(lactic-co-glycolic)acid (PLGA) copolymer. The ratio of lactic to glycolic acid monomers in the polymer can be about 50/50 weight percentage. Additionally, the PLGA copolymer can be about 20 to about 90 weight percent of the bioerodible implant. Alternately, the PLGA copolymer can be about 40 percent by weight of the bioerodible implant.

The present invention provides biodegradable ocular implants and methods for treating medical conditions of the eye. Usually, the implants are formed to be monolithic, i.e., the particles of active agent are distributed throughout the biodegradable polymer matrix. Furthermore, the implants are formed to release an active agent into an ocular region of the eye over various time periods. The active agent can be released over a time period including, but is not limited to, approximately twelve months, ten months, nine months, eight months, six months, seven months, eight months, three months, one month, or less than one month.

### Biodegradable Implants For Treating Medical Conditions of the Eye

The implants of the invention include an active agent dispersed within a biodegradable polymer. In some embodiments, the anti-inflammatory agent is a steroidal anti-inflammatory agent, such as a corticosteroid such as dexamethasone. In some embodiments, dexamethasone is the only active agent present in the implant.

The steroidal anti-inflammatory agent, such as dexamethasone, can constitute from about 10% to about 90% by weight of the implant. In one variation, the agent is from about 40% to about 80% by weight of the implant. In a preferred variation, the agent comprises about 60% by weight of the implant.

### The Biodegradable Polymer Matrix

In one variation, the active agent may be homogeneously dispersed in the biodegradable polymer matrix of the implants. The selection of the biodegradable polymer matrix to be employed will vary with the desired release kinetics, patient tolerance, the nature of the disease to be treated, and the like. Polymer characteristics that are considered include, but are not limited to, the biocompatibility and biodegradability at the site of implantation, compatibility with the active agent of interest, and processing temperatures. The biodegradable polymer matrix usually comprises at least about 10, at least about 20, at least about 30, at least about 40, at least about 50, at least about 60, at least about 70, at least about 80, or at least about 90 weight percent of the implant. In one variation, the biodegradable polymer matrix comprises about 40% by weight of the implant.

Biodegradable polymer matrices which may be employed include, but are not limited to, polymers made of monomers such as organic esters or ethers, which when degraded result in physiologically acceptable degradation products. Anhydrides, amides, orthoesters, or the like, by themselves or in combination with other monomers, may also be used. The polymers are generally condensation polymers. The polymers may be crosslinked or non-crosslinked. If crosslinked, they are usually not more than lightly crosslinked, and are less than 5% crosslinked, usually less than 1% crosslinked.

Of particular interest are polymers of hydroxyaliphatic carboxylic acids, either homo- or copolymers, and polysaccharides. Included among the polyesters of interest are homo- or copolymers of D-lactic acid, L-lactic acid, racemic lactic acid, glycolic acid, caprolactone, and combinations thereof. Copolymers of glycolic and lactic acid are of particular interest, where the rate of biodegradation is controlled by the ratio of glycolic to lactic acid. The percent of each monomer in poly(lactic-co-glycolic)acid (PLGA) copolymer may be 0-100%, about 15-85%, about 25-75%, or about 35-65%. In a preferred variation, a 50/50 PLGA copolymer is used . More preferably, a random copolymer of 50/50 PLGA is used.

Biodegradable polymer matrices that include mixtures of hydrophilic and hydrophobic ended PLGA may also be employed, and are useful in modulating polymer matrix degradation rates. Hydrophobic ended (also referred to as capped or end-capped) PLGA has an ester linkage hydrophobic in nature at the polymer terminus. Typical hydrophobic end groups include, but are not limited to alkyl esters and aromatic esters. Hydrophilic ended (also referred to as uncapped) PLGA has an end group hydrophilic in nature at the polymer terminus. Examples of suitable hydrophilic end groups that may be incorporated to enhance hydrolysis include, but are not limited to, carboxyl, hydroxyl, and polyethylene glycol. The specific end group will typically result from the initiator employed in the polymerization process. For example, if the initiator is water or carboxylic acid, the resulting end groups will be carboxyl and hydroxyl. Similarly, if the initiator is a monofunctional alcohol, the resulting end groups will be ester or hydroxyl.

The implants may be formed from all hydrophilic end PLGA or all hydrophobic end PLGA. In general, however, the ratio of hydrophilic end to hydrophobic end PLGA in the biodegradable polymer matrices of this invention range from about 10:1 to about 1:10 by weight. For example, the ratio may be 3:1, 2:1, or 1:1 by weight. In a preferred variation, an implant with a ratio of hydrophilic end to hydrophobic end PLGA of 3:1 w/w is used.

### Additional Agents

Other agents may be employed in the formulation for a variety of purposes. For example, buffering agents and preservatives may be employed. Preservatives which may be used include, but are not limited to, sodium bisulfite, sodium bisulfate, sodium thiosulfate, benzalkonium chloride, chlorobutanol, thimerosal, phenylmercuric acetate, phenylmercuric nitrate, methylparaben, polyvinyl alcohol and phenylethyl alcohol. Examples of buffering agents that may be employed include, but are not limited to, sodium carbonate, sodium borate, sodium phosphate, sodium acetate, sodium bicarbonate, and the like, as approved by the FDA for the desired route of administration. Electrolytes such as sodium chloride and potassium chloride may also be included in the formulation.

The biodegradable ocular implants may also include additional hydrophilic or hydrophobic compounds that accelerate or retard release of the active agent. Furthermore, the inventors believe that because hydrophilic end PLGA has a higher degradation rate than hydrophobic end PLGA due to its ability to take up water more readily, increasing the amount of hydrophilic end PLGA in the implant polymer matrix will result in faster dissolution rates. Figure 9 shows that the time from implantation to significant release of active agent (lag time) increases with decreasing amounts of hydrophilic end PLGA in the ocular implant. In Figure 9, the lag time for implants having 0% hydrophilic end PLGA (40% w/w hydrophobic end) was shown to be about 21 days. In comparison, a significant reduction in lag time was seen with implants having 10% w/w and 20% w/w hydrophilic end PLGA.

### Applications

Examples of medical conditions of the eye which may be treated by the implants and methods of the invention include, but are not limited to, uveitis, macular edema, diabetic macular edema, macular degeneration, retinal detachment, ocular tumors, fungal or viral infections, multifocal choroiditis, diabetic retinopathy, proliferative vitreoretinopathy (PVR), sympathetic opthalmia, Vogt Koyanagi-Harada (VKH) syndrome, histoplasmosis, uveal diffusion, and vascular occlusion. In one variation, the implants are particularly useful in treating such medical conditions as uveitis, macular edema, vascular occlusive conditions, proliferative vitreoretinopathy (PVR), and various other retinopathies.

### Method of Implantation

The biodegradable implants may be inserted into the eye by a variety of methods, including placement by forceps, by trocar, or by other types of applicators, after making an incision in the sclera. In some instances, a trocar or applicator may be used without creating an incision. In a variation, a hand held applicator is used to insert one or more biodegradable implants into the eye. The hand held applicator typically comprises an 18-30 GA stainless steel needle, a lever, an actuator, and a plunger.

The method of implantation generally first involves accessing the target area within the ocular region with the needle. Once within the target area, e.g., the vitreous cavity, the lever on the hand held device is depressed to cause the actuator to drive the plunger forward. As the plunger moves forward, it pushes the implant into the target area.

According to an embodiment, the long axis of an applicator having a needle having a bevel can be held parallel to the limbus, and the sclera can be engaged at an oblique angle with the bevel of the needle upwards (away from the sclera) to create a shelved scleral path. The tip of the needle can then be advanced within the sclera for about 1 mm (parallel to the limbus), then re-directed toward the center or the eye and advanced until penetration of the sclera is completed and the vitreous cavity of a patient's eye is entered. After that, the applicator can be activated to deliver a biodegradable implant within the vitreous of the patient.

### Methods of Treatment of DME

In an embodiment, a method of treating an ocular condition, for example, diabetic macular edema comprises administering to the eye of a patient in need thereof, at a frequency between once every six months and once a year, a bioerodible implant comprising an active ingredient, such as dexamethasone, and a biodegradable polymer matrix. The bioerodible implant can be of the types disclosed herein.

According to some embodiments, the administration of the implant to the eye of the patient can include injection of the implant into to the eye of a patient in need thereof. According to some embodiments, the method of treating an ocular condition can include injecting a bioerodible implant into the vitreous, anterior chamber, subconjunctival space, or any other suitable area of the eye.

The methods can be used to treat certain ocular conditions, including those related to ischemic retinopathy, neovascular retinopathy, or both ischemic retinopathy and neovascular retinopathy. Some conditions related to ischemic retinopathy, that can be treated by methods disclosed herein, can include diabetic macular edema, central vein occlusion, and branched vein occlusion. Some conditions related to neovascular retinopathy, that can be treated by methods disclosed herein, can include proliferative diabetic retinopathy, exudative age-related macular degeneration, pathological myopia, choroidal neovascularation, secondary to histoplasmosis, polypoidal choroidal neovasularization, and retinal angiomatous proliferation, The method may be used to treat age-related macular degeneration, diabetic macular edema, pathological myopia branch retinal vein occlusion, and central retinal vein occlusion.

To "treat," as used here, means to deal with medically. It includes, for example, administering the bioerodible implant of the invention to prevent the onset of DME as well as to alleviate its severity.

In one embodiment, the bioerodible implant is administered once every 6 months to the eye of a patient in need thereof to treat DME In another embodiment, the implant is administered once every 4 months, 5 months, 7 months, 8 months, 9 months, 10 months, 11 months, or every 12 months (or year). In some embodiments, the bioerodible implant is administered once every 4 months to 12 months, every 5 months to 10 months, every 6 months to 9 months, or every 8 months to 12 months to the eye of a patient in need thereof to treat DME In some embodiments, the bioerodible implant is administered at one or more of the frequencies described above for the remainder of the lifetime of the patient. In other embodiments, the bioerodible implant is administered at one or more of the frequencies described above for a period of time of 2 years, 3 years, 4 years, 5 years, 10 years, 15 years, for the lifetime of the patient, or until the ocular condition (such as DME) is adequately treated. Such methods at such dosing regimens described above can be therapeutically effective to treat DME in a patient in need thereof. In some embodiments, the methods described herein can increase the visual acuity of a patient having DME

Since the implant is biodegradable, subsequent implant(s) can be inserted without the need for surgical removal of the existing implant. By avoiding the peak vitreous drug concentrations produced by the need for frequent repeat injections, the implant may potentially reduce the risk of unwanted side effects, such as cataract formation, IOP elevation, and glaucoma and it may reduce the risk of injection-related complications, such as lens injury, retinal detachment, and infectious endophthalmitis.

According to some embodiments, the bioerodible implant can treat an ocular condition in a patient having macular edema, such as diabetic macular edema independent of the lens status of the patient. For example, in some embodiments, the treatment of DME can be achieved using the implants and methods disclosed herein regardless of whether a patient has a phakic lens or a pseudophakic lens. According to some embodiments, treatment of DME can be achieved in patients having a pseudophakic lens. According to some embodiments, treatment of DME can be achieved in patients having a phakic lens, but who are scheduled for or plan to have cataract surgery.

According to some embodiments, the bioerodible implant can treat an ocular condition in a patient who is refractory to other existing treatments for DME For example, according to some methods, a patient having DME who is refractive to anti-VEGF intraocular injections can effectively be treated by the methods disclosed herein. According to some other methods, a patient having DME who is refractive to laser photocoagulation can effectively be treated by the methods disclosed herein.

### EXAMPLES

The following examples are provided for the purposes of further describing the embodiments described herein, and do not limit the scope of the invention.

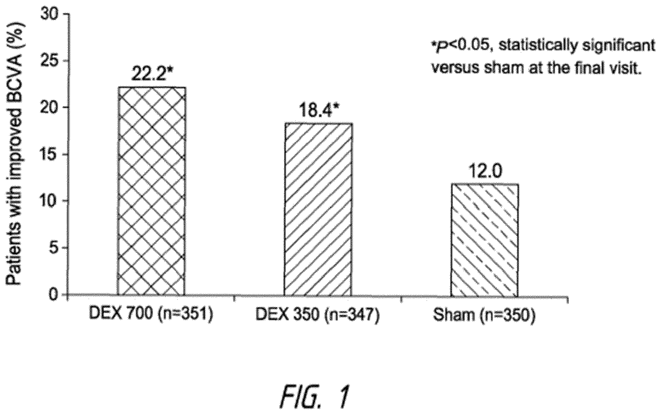

A Phase III, multicenter, masked, randomized, sham-controlled trial was conducted to assess the safety and efficacy of 700 µg and 350 µg dexamethasone posterior segment drug delivery system in patients with diabetic macular edema ("DME"). The study was conducted over a period of three years. The results of these studies are shown in Figures 1-7.

The implants used in the study were comprised of dexamethasone and a polymer matrix of 50:50 poly (D,L-lactide-co-glycolide) PLGA, constituted of two grades of PLGA (50:50 PLGA ester and 50:50 PLGA acid). See Table 1 for details. The two PLGAs combination as presented in Table 2 was chosen for the biodegradable polymer matrix. General properties of the chosen PLGAs are presented in Table 3.

**Table 1: Qualitative composition of a sample DEX PS DDS**

| **Component** | **Quality Standard** | **Function** |
|---|---|---|
| Dexamethasone | Ph. Eur. | Active ingredient |
| 50:50 PLGA ester | Allergan, Inc. | Biodegradable extended release polymer matrix |
| 50:50 PLGA acid | Allergan, Inc. | Biodegradable extended release polymer matrix |

**Table 2: Quantitative Composition of a sample DEX PS DDS (manufacturing batch formula)**

| **Component** | | **350 µg** | **700 µg** |
|---|---|---|---|
| | | | |
| Dexamethasone | | 350 µg (60%) | 700 µg (60%) |
| 50:50 PLGA ester (hydrophobic) | | 58 µg (10%) | 116 µg (10%) |
| 50:50 PLGA acid (hydrophilic) | | 175 µg (30%) | 350µg (30%) |

**Table 3 General properties of PLGAs**

| | **50:50 PLGA ester** | **50:50 PLGA acid** |
|---|---|---|
| Common Names | Resomer RG 502, PLG, PLGA, Poly (lactic-glycolic) acid, 50:50 Poly (D,L-lactide-co-glycolide), Polylactic/Polyglycolic acid, Polyglactin 910 | Resomer RG 502H, PLG acid end, PLGA acid end, 50:50 Poly (D,L-lactide-co-glycolide) acid end |
| Structure | 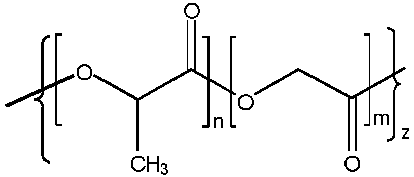 | 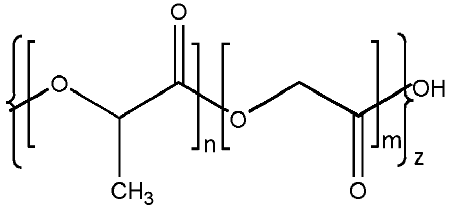 |
| | Where: | Where: |
| | n=m | n=m |
| | n = number of lactide repeating units | n = number of lactide repeating units |
| | m = number of glycolide repeating units | m = number of glycolide repeating units |
| | z = overall number of lactide-co-glycolide repeating units | z = overall number of lactide-co-glycolide repeating units |
| CAS Number | 34346-01-5 | 26780-50-7 |
| Empirical Formula | [(C3H4O2)x. (C2H2O2)y]CH3, x:y=50:50 | [(C3H4O2)x. (C2H2O2)y]OH, x:y=50:50 |
| Description | white to off white powder | white to near white powder |

In the trial, patients having DME received either a bioerodible implant having 700 µg of dexamethasone, a bioerodible implant having 350 µg of dexamethasone, or a bioerodible implant having 0 µg of dexamethasone (a sham). The implants were injected into the vitreous of one eye of each patient. The patients were evaluated for retreatment every 3 months after a month 6 visit. Retreatment (i.e. administration of another implant) was allowed every 6 months. Retreatment was allowed if central retinal thickness in the patient was greater than 175 µm or if there was any evidence of residual retinal edema. Patients were allowed a maximum of 7 implants over the three-year study period per eye.

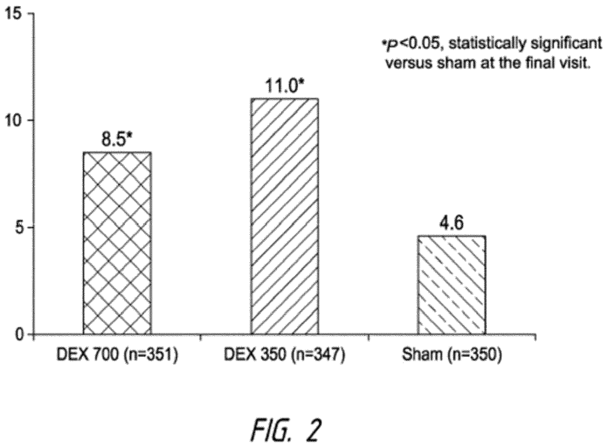

As illustrated in Figures 1-2, a statistically significant improvement in Best Corrected Visual Acuity ("BCVA") score was achieved in patients receiving the 700 µg dexamethasone implant and in patients receiving the 350 µg dexamethasone implant compared to patients receiving the sham implant. Figure 1 illustrates that 22.2% of patients receiving the 700 µg dexamethasone implant demonstrated a BCVA improvement greater than or equal to 15 letters and that 18.4% of patients receiving the 350 µg dexamethasone implant demonstrated a BCVA improvement greater than or equal to 15 letters. Figure 2 illustrates that 8.5% of patients receiving the 700 µg dexamethasone implant demonstrated a BCVA improvement greater than or equal to 20 letters and that 11.0% of patients receiving the 350 µg dexamethasone implant demonstrated a BCVA improvement greater than or equal to 20 letters.

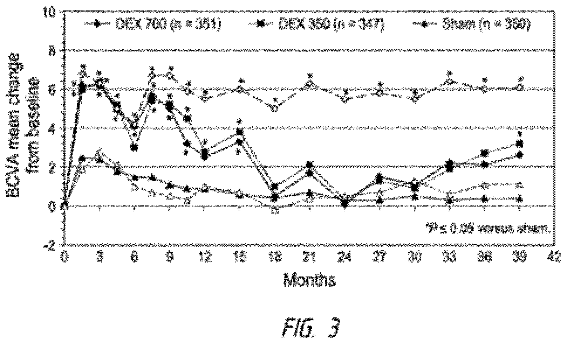

Figure 3 illustrates that patients in groups receiving the 700 µg and 350 µg dexamethasone-containing implants generally showed a greater improvement in BCVA over the three-year study period than the patients receiving the sham implant. As shown in Figure 3, a rapid increase in BCVA was observed in patients receiving the dexamethasone implants. Specifically, a mean BCVA increase of about 6 letters from baseline was observed over the first about 3 months of treatment for patients receiving the 350 µg dexamethasone implant, and a mean BCVA increase of about 7 letters from baseline was observed over the first about 3 months of treatment for patients receiving the 700 µg dexamethasone implant.

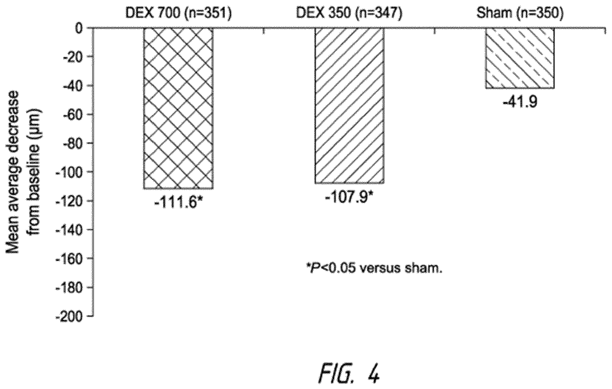

As illustrated in Figure 4, the mean average decrease from baseline in Central Subfield Retinal Thickness ("CSRT") was greater in patients receiving the dexamethasone implants than in patients receiving the sham implant. Figure 4 illustrates that patients receiving the 700 µg dexamethasone implant demonstrated a CSRT mean average decrease of 111.6 µm from baseline and that patients receiving the 350 µg dexamethasone implant demonstrated a CSRT mean average decrease of 107.9 µm from baseline.

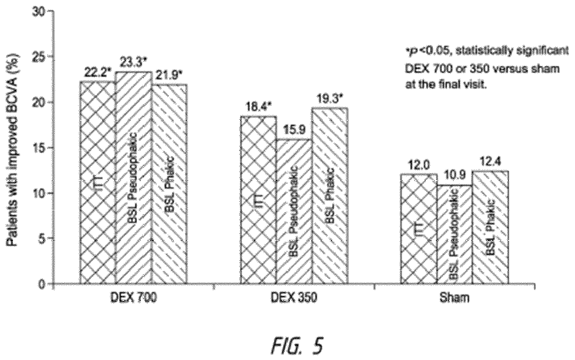

As illustrated in Figure 5, the dexamethasone implants led to significant BCVA improvements, regardless of the lens status of the patient at baseline.

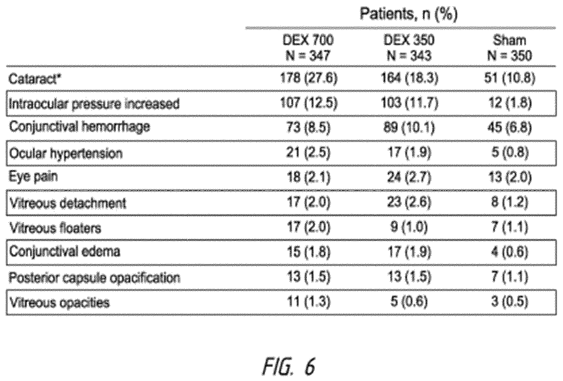

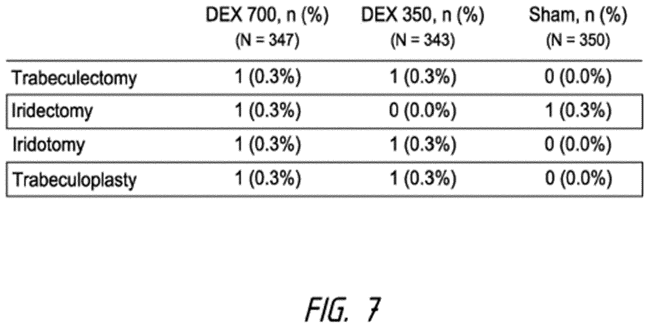

The adverse event profile from the study is shown below in Figure 6. As shown in the Figure, the most common adverse events experienced in the study were cataracts and intraocular pressure. However, despite the occurrence of the adverse event of increased IOP, surprisingly, very few patients (about 0.3% per surgery type per study group) underwent surgery during the study for management of IOP. The number of patients who underwent surgery and the type of surgeries underwent are illustrated in Figure 7.

As shown from the data in the Figures, the implants and methods disclosed herein resulted in significant, long term improvement in vision in patients with diabetic macular edema. The proportion of patients with a greater than or equal to 15-letter gain was significantly higher with the 350 µg and 700 µg dexamethasone implants compared with sham at Year 3. The treatment benefit was observed with a mean of 4.1 injections over 3 years.

Table 4 below illustrates the visual acuity outcomes at Month 39 of the study.

**Table 4**

| **Study** | **Outcomes** | **OZURDEX®** | **Sham** | **Estimated Difference (95% CI)** |
|---|---|---|---|---|
| 1^{a} | Mean (SD) Baseline BCVA (Letters) | 56 (10) | 57 (9) | |
| | Median (range) Baseline BCVA (Letters) | 59 (34-95) | 58 (34-74) | |
| | Gain of≥15 letters in BCVA (n(%)) | 34 (21%) | 19 (12%) | 9.3% (1.4%, 17.3%) |
| | Loss of≥15 letters in BCVA (n(%)) | 15 (9%) | 17 (10%) | -1.1% (-7.5%, 5.3%) |
| | Mean change in BCVA (SD) | 4.1 (13.9) | 0.9 (11.9) | 3.2 (0.4, 5.9) |
| | Mean (SD) Baseline BCVA (Letters) | 55 (10) | 56 (9) | |
| 2^{b} | Median (range) Baseline BCVA (Letters) | 58 (34-72) | 58 (36-82) | |
| | Gain of ≥15 letters in BCVA (n(%)) | 30 (18%) | 16 (10%) | 8.4% (0.9%, 15.8%) |
| | Loss of≥15 letters in BCVA (n(%)) | 30 (18%) | 18 (11%) | 7.1% (-0.5%, 14.7%) |
| | Mean change in BCVA (SD) | 0.4 (17.5) | 0.8 (13.6) | -0.7 (-4.1, 2.6) |

| | | | | |
|---|---|---|---|---|
| ^{a}Study 1: **OZURDEX®,** N=163: Sham, N=165 ^{b}Study 2: **OZURDEX®,** N=165: Sham, N=163 ^{c}14% (16.8% from **OZURDEX®** and 12.2% from Sham) of patients had BCVA outcome at Month 39. for the remaining patients, the data at Month 36 or earlier was carried forward. | | | | |

Table 5 below illustrates the best corrected visual acuity outcomes for the pseudophakic and phakic subgroups.

**Table 5**

| **Subgroup (Pooled)** | **Outcomes** | **OZURDEX®** | **Sham** | **Estimated Difference (95% CI)** |
|---|---|---|---|---|
| ^{a}Pseudophakic | Gain of ≥15 letters in BCVA (n(%)) | 16 (20%) | 11 (11%) | 8.4% (-2.2%, 19.0%) |
| | Loss of≥15 letters in BCVA (n(%)) | 4 (5%) | 7 (7%) | -2.2% (-9.1%, 4.7%) |
| | Mean change in BCVA (SD) | 5.8 (11.6) | 1.4 (12.3) | 4.2 (0.8, 7.6) |
| ^{b}Phakic | Gain of ≥ 15 letters in BCVA (n(%)) | 48 (20%) | 24 (11%) | 9.0% (2.7%, 15.4%) |
| | Loss of≥15 letters in BCVA (n(%)) | 41 (17%) | 28 (12%) | 4.4% (-1.9%, 10.7%) |
| | Mean change in BCVA (SD) | 1.0 (16.9) | 0.6 (12.9) | 0.3 (-2.4, 3.0) |

| | | | | |
|---|---|---|---|---|
| ^{a}Pseudophakic: **OZURDEX®**, N=82: Sham, N=99 ^{b}Phakic: **OZURDEX®**, N=246; Sham, N=229 ^{c}14% (16.8% from **OZURDEX®** and 12.2% from Sham) of patients had BCVA outcome at Month 39, for the remaining patients data at Month 36 or earlier was used in the analysis. | | | | |

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In addition while the number of variations of the invention have been shown and described in detail, other modifications, which are within the scope of this invention, will be readily apparent to those of skill in the art based on this disclosure. It is also contemplated that various combinations or subcombinations of the specific features and aspects of the embodiments can be made and still fall within the scope of the invention. Accordingly, it should be understood that various features and aspects of the disclosed embodiments can be combined with, or substituted for, one another in order to perform varying modes of the disclosed invention. Thus, it is intended that the scope of the present invention herein disclosed should not be limited by the particular disclosed embodiments described above, but should be determined only by a fair reading of the claims.

### Items:

1. A method for treating diabetic macular edema (DME), the method comprising injecting a bioerodible implant into the vitreous of a human at a frequency of once every about six months to once every about nine months, the bioerodible implant comprising a continuous, double extruded rod comprising dexamethasone homogeneously dispersed within a biodegradable polymer matrix; wherein
   the biodegradable polymer matrix comprises a mixture of poly(D,L-lactide-co-glycolide) (PLGA) having hydrophilic end groups and poly(D,L-lactide-co-glycolide) (PLGA) having hydrophobic end groups; and
   wherein the bioerodible implant is sized for implantation in the vitreous of the human; and wherein the method is therapeutically effective to treat DME
2. The method of item 1, wherein the human is refractory to anti-VEGF treatment for DME
3. The method of item 1 or item 2, wherein the dexamethasone is present in the bioerodible implant in an amount of 60% by weight, based on the total weight of the bioerodible implant.
4. The method of any one of items 1-3, wherein the PLGA having hydrophobic end groups is present in the bioerodible implant in an amount of 10% by weight, based on the total weight of the bioerodible implant.
5. The method of any one of items 1-4, wherein the PLGA having hydrophilic end groups is present in the bioerodible implant in an amount of 30% by weight, based on the total weight of the bioerodible implant.
6. The method of any one of items 1-5, wherein the human has a pseudophakic lens.
7. The method of any one of items 1-5, wherein the human has a phakic lens.

## Claims

1. A bioerodible implant for use in a method for treating diabetic macular edema (DME), the method comprising injecting a bioerodible implant into the vitreous of a human at a frequency of once every about 6 months to once every about 12 months, the bioerodible implant comprising a continuous, double extruded rod comprising dexamethasone homogeneously dispersed within a biodegradable polymer matrix; wherein
the biodegradable polymer matrix comprises a mixture of poly(D,L-lactide-co-glycolide) (PLGA) having hydrophilic end groups and poly(D,L-lactide-co-glycolide) (PLGA) having hydrophobic end groups; and
wherein the bioerodible implant is sized for implantation in the vitreous of the human; and wherein the method is therapeutically effective to treat DME

2. The method of Claim 1, wherein the human is refractory to anti-VEGF treatment for DME.

3. The method of Claim 1 or Claim 2, wherein the dexamethasone is present in the bioerodible implant in an amount of 60% by weight, based on the total weight of the bioerodible implant.

4. The method of any one of Claims 1-3, wherein the PLGA having hydrophobic end groups is present in the bioerodible implant in an amount of 10% by weight, based on the total weight of the bioerodible implant.

5. The method of any one of Claims 1-4, wherein the PLGA having hydrophilic end groups is present in the bioerodible implant in an amount of 30% by weight, based on the total weight of the bioerodible implant.

6. The method of any one of Claims 1-5, wherein the human has a pseudophakic lens.

7. The method of any one of Claims 1-5, wherein the human has a phakic lens.
